Europäisches Patentamt

**(19)** European Patent Office

Office européen des brevets

**(11)** Publication number : **0 523 092 B1**

**(12)** # EUROPEAN PATENT SPECIFICATION

**(45)** Date of publication of patent specification : **29.06.94 Bulletin 94/26**

**(51)** Int. Cl.$^5$ : **G01N 1/02**

**(21)** Application number : **91906727.2**

**(22)** Date of filing : **02.04.91**

**(86)** International application number : **PCT/CA91/00108**

**(87)** International publication number : **WO 91/15745 17.10.91 Gazette 91/24**

**(54) METHOD AND DEVICE FOR SOLID PHASE MICROEXTRACTION AND DESORPTION.**

**(30)** Priority : **02.04.90 GB 9007356**

**(43)** Date of publication of application : **20.01.93 Bulletin 93/03**

**(45)** Publication of the grant of the patent : **29.06.94 Bulletin 94/26**

**(84)** Designated Contracting States : **AT BE CH DE DK ES FR GB GR IT LI LU NL SE**

**(56)** References cited :
EP-A- 0 159 230
FR-A- 2 648 227
US-A- 3 797 318
ANALYTICAL CHEMISTRY, vol. 62, no. 19, October 1, 1990, COLUMBUS US pages 2145-2148; C.L. ARTHUR et al.: "Solid Phase Microextraction with Thermal Desorption Using Fused Silica Optical Fibers", see page 2145; figure 1

**(56)** References cited :
WATER POLLUTION RESEARCH JOURNAL OF CANADA vol. 24, no. 1, 1989, CA pages 179-191; BELARDI et al.: "The Application of Chemically Modified Fused Silica Fibers in the Extraction of Organics from Water Matrix Samples", see page 180, line 17 - page 185, line 10, see page 190, lines 6-12
ANALYTICAL CHEMISTRY, vol. 59, no. 10, May 15, 1987, COLUMBUS, US pages 1475-1478; J. PAWLISZYN et al.: "Sample Introduction for Capillary Gas Chromatography with Laser Descorption and Optical Fibers", see page 1476, left column, line 18 - right column, line 43

**(73)** Proprietor : **PAWLISZYN, Janusz B.**
383 Dunvegan Drive
Waterloo, Ontario N2K 1W7 (CA)

**(72)** Inventor : **PAWLISZYN, Janusz B.**
383 Dunvegan Drive
Waterloo, Ontario N2K 1W7 (CA)

**(74)** Representative : **Warren, Anthony Robert et al**
BARON & WARREN
18 South End
Kensington
London W8 5BU (GB)

EP 0 523 092 B1

## Description

This invention relates to a method and device for solid phase microextraction and analysis and, in particular, relates to microextraction and analysis being carried out using various types of a single fiber which can be coated with various materials or uncoated.

Presently, in the organic analysis of environmental samples which involve the separation of components of interest from such matrices as soil, water, fly ash, tissue or other material, liquid extraction is tradionally used as the separation process. For example, water samples are usually extracted with organic solvent. Similarly, solid samples are leeched with an organic solvent in a SOXHLET apparatus. Methods based on solvent extraction are often time consuming, difficult to automate and are very expensive since they require high purity organic solvents and these organic solvents are expensive to dispose of. Further, the organic solids usually have high toxicity and are difficult to work with. In addition, the extraction processes can be highly non-selective. Therefore, sequential chromatographic techniques must sometimes be used to separate complex mixtures after extraction, significantly increasing the overall analysis time and the cost. EP-A1-159 230 discloses an extraction method of components in a liquid by placing packets of fibers in contact with said liquid in extracting the components.

Solid phase extraction is a known effective alternative to liquid-liquid extraction in the analysis aqueous samples. The primary advantage of solid phase extraction is the reduced consumption of high purity solvents and the resulting reduction in laboratory costs and the costs of solvent disposal. Solid phase extraction also reduces the time required to isolate the analyte of interest. However, solid phase extraction continues to use solvents and often suffers from high blank values. Further, there is considerable variation between the products offered by different manufacturers and lot-to-lot variation can be a problem when carrying out solid phase extraction procedures. Solid phase extraction cartridges available for manufacturers are normally constructed of plastic which can adsorb the analyte and increase interferences in the analysis. The disposable plastic cartridges used in the solid phase extraction process are first activated using organic solvent. The excess organic solvent is then removed and the sample to be tested is passed through the cartridge. The organic components from the sample are adsorbed on the chemically modified silica surface of the material in the cartridge. Both molecules of interest as well as interferences are retained on the cartridge material. During desorption, a selective solvent is chosen to first remove the interferences. The analyte is then washed out of the cartridge. The analytical procedure from that point is identical to that used in liquid-liquid extraction. The analyte is first preconcentrated and the mixture is then injected into an appropriate high resolution chromatographic instrument. Steps involving the use of organic solvents are the most time consuming.

According to one aspect of the present invention, a device for carrying out solid phase microextraction with components contained in a fluid carrier is characterized by, in combination a fiber and a housing surrounding said fiber, said housing containing access means so that said carrier and components can be brought into contact with said fiber.

According to another aspect of the present invention, there is provided a method of carrying out solid phase microextraction and analysis with components contained in a carrier using a fiber, said method being characterized by placing said fiber in contact with said carrier containing said components for a sufficient period of time for extraction to occur, subsequently removing said fiber from said carrier and placing the fiber into a suitable analytical instrument and carrying out thermal desorption with respect to at least one component on said fiber.

According to a further aspect of the present invention, there is provided a method of carrying out solid phase microextraction and analysis with components contained in a carrier using a fiber contained in a housing, said housing having access means so that said carrier can be brought into contact with said fiber, said method being characterized by contacting said fiber with said carrier for a sufficient time to allow microextraction to occur, ending said contact and placing said fiber in a suitable analytical instrument in such a manner that desorption occurs with respect to at least one component on said fiber.

Reference will now be made to the accompanying drawings, in which:-

Figure 1 is a partial sectional side view of a syringe and fiber with the plunger depressed;

Figure 2 is a schematic side view of a slightly different syringe and fiber with the plunger withdrawn;

Figure 3 is a schematic side view of a needle portion of a syringe containing a hollow fiber;

Figure 4 is a graph of amount of analyte extracted versus time;

Figure 5 is a graph showing the results of a typical gas chromatography analysis;

Figure 6 is a graph showing another analysis from a gas chromatograph;

Figure 7a shows a chromatogram produced when using the solid phase microextraction embodying the present invention;

Figure 7b shows a chromatogram produced when using the prior art method of liquid-liquid extraction for the same components as those of Figure 7a;

Figure 8 is a chromatogram of the extraction of gasoline components from water with silicone coated fibers; and

Figure 9 is a chromatogram from the extraction of organics from coal gasification waste water using a silicone coated fiber.

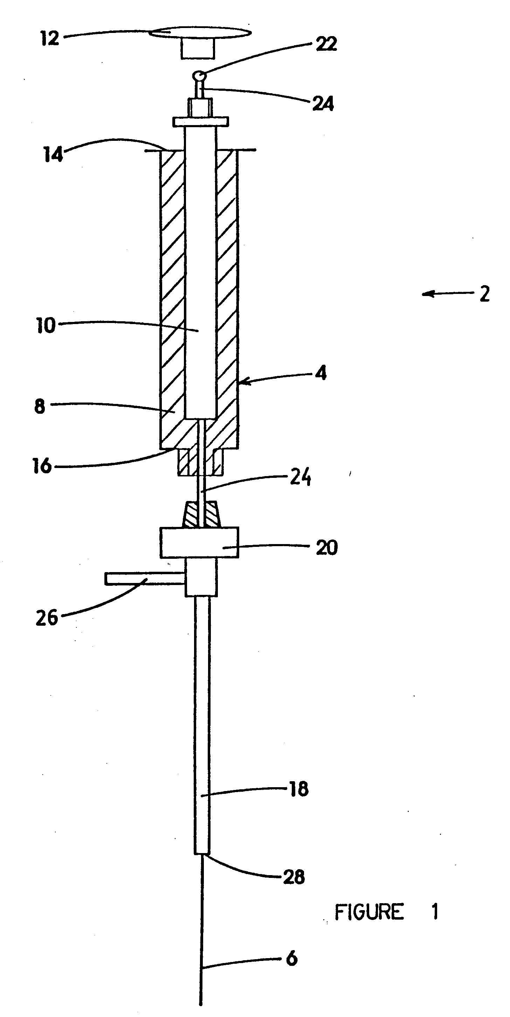

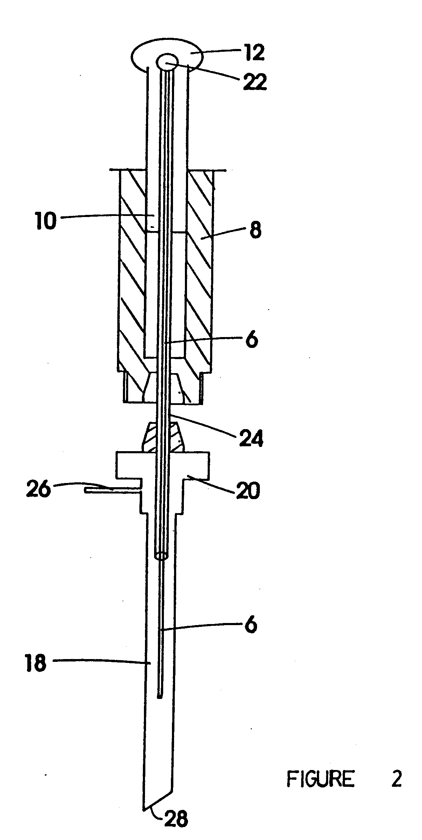

Referring to Figures 1 and 2 in greater detail, a device 2 for carrying out solid phase microextraction has a syringe 4 containing a fiber 6. The syringe 4 is made up of a barrel 8 which contains a plunger 10 and is slidable within the barrel 8. The plunger 10 has a handle 12 extending from one end 14 of the barrel 8. At the opposite end 16 of the barrel 8, there is located a needle 18 which is connected to the end 16 by the connector 20. The handle 12 and the needle 18 and connector 20 are shown in an exploded position relative to the barrel 8 for ease of illustration.

The fiber 6 is a solid thread-like material that extends from the needle 18 through the barrel 8 and out the end 14. An end of the fiber 6 (not shown) located adjacent to the handle 12 has retention means 22 located thereon so that the fiber will move longitudinally as the plunger 10 slides within the barrel 8. The retention means can be simply a drop of epoxy which is placed on the end of the fiber 6 near the handle 12 and allowed to harden. The fiber 6 is partially enclosed in a metal sleeve 24 which surrounds that portion of the fiber 6 located within the plunger 10, the barrel 8 and part of the needle 18. The purpose of the metal sleeve 24 is to protect the fiber 6 from damage and to ensure a good seal during operation of the device. Extending from the connector 20 is an optional inlet 26. The purpose of the inlet 26 is to allow alternate access to the fiber. For example, when the fiber is contained within the needle 18, fluid could contact the fiber 6 by entering the inlet 26 and exiting from a free end 28 of the needle 18. The inlet 26 can also be used to contact the fiber with an activating solvent.

In Figure 2, a schematic version of the device 2 is shown. The plunger is in a withdrawn position and the free end of the fiber 6 is located entirely within the needle 18. The access permitted by the inlet 26 when the fiber is in the position shown in Figure 2 can readily be understood. Obviously, fluid contacting the fiber 6 within the needle 18 could also enter the free end 28 of the needle 18 and exit from the access 26.

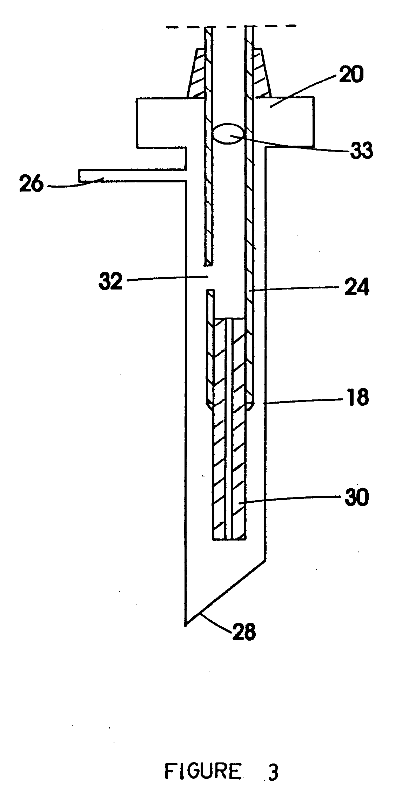

In Figure 3, only the needle portion of the device is shown. A fiber 30 extending from the metal sleeve 24 is hollow. It can be seen that there is an opening 32 in the wall of the metal sleeve 24 to allow access to an interior of the sleeve 24 as well as an interior of the fiber 30. For example, fluid could enter the inlet 26 and an interior of the needle 18. Then, the fluid could pass through the opening 32 and through an interior of the fiber 30 and ultimately exit from the free end 28 of the needle 18. In this embodiment, the fiber does not extend to the handle 12 (not shown) but only the metal sleeve 24 extends to the handle 12. The fiber 30 can still be moved beyond the end 28 of the needle 18 by depressing the plunger and returned to the position shown in Figure 3 by moving the plunger to the withdrawn position.

Alternatively, if it is desired to have the fiber 30 located within the needle 18 at all times, contact with the fiber 30 can be attained through the inlet 26 or the opening 32 and the free end 28. A plug 33 located within the metal sleeve 24 prevents any fluid from travelling up the sleeve to the handle. In some situations, the fluid could flow through the sleeve 24.

In general terms, the syringe could be said to be a housing for the fibers 6, 30 and the access means could be the action of the plunger 10 in moving the fiber beyond the end 28 or, alternatively, the access means could be the inlet 26.

The disadvantages and inconveniences of the previous processes for analyzing various fluids are overcome by the solid phase microextraction technique of the present invention. The diameter of the fibers will vary but will preferably be between 0.05 millimeters and 1 millimeter. Much of the experimentation on which the present invention was based, was carried out using fused silica fibers that were chemically modified. The fused silica fibers are widely used in optical communication and are often referred to as optical fibers.

Chemical modification of these fibers can be achieved by the preparation of the surface involving etching procedures to increase the surface area followed by chemical attachment of the desired coating. The stationary phases bonded to the surface of the silica fibers are similar to that used in fused silica gas chromatograph columns or high performance liquid chromatography columns.

As an example, fused silica fibers were obtained from Polymicro Technologies Inc., Phoenix, Arizona and these fibers were coated with polyimide and had an outer diameter of approximately 171 $\mu$m. Uncoated fused silica was obtained by burning off the polyimide coating and gently scraping off the charred portion. To use the polyimide film as a stationary phase, it was first heated at 350° C for four hours. The polyimide was then burned off and the char removed, except for a one to two millimeter portion at the end of the fiber. In all cases, the polyimide was burned off after the fiber had been inserted into the syringe and trimmed to the correct length. After burning, the fiber became fragile and had to be handled carefully. The metal casing is used to strengthen the fiber. The normal lifetime for a prepared fiber was five to six weeks with regular use.

The solid phase microextraction process does not require a sophisticated coating system to be a useful technique. Either the uncoated fiber, fused silica, silicone or the polyimide films that optical fibers are shipped with can be a suitable stationary phase.

The method of solid phase microextraction and analysis consists of a few simple steps. For example, when a water matrix sample containing components of interest is desired to be analyzed, the plunger of the syringe is depressed and the exposed fiber extending from the free end of the needle is inserted into the water matrix sample. The organic components of the water are extracted into the non-polar phase. Water is considered to be the carrier in a water matrix sample. Where the water sample is contained in a bottle containing a septum, the needle is inserted through the septum first before the plunger is depressed so that the fiber will not be damaged by the septum. When the microextraction has occurred to a sufficient degree (usually approximately two minutes), the plunger is moved to the withdrawn position causing the fiber to be drawn into the needle and the needle is removed from the sample bottle through the septum. Preferably, the sample is stirred while the fiber is inserted. The time for extraction will depend on many factors including the components being extracted as well as the thickness and type of coating, if any, on the fiber. Usually, the extraction time is approximately two minutes. The plunger is then moved to the withdrawn position to retract the fiber into the needle. The needle is then removed from the bottle and is inserted through the septum in an injection port of a conventional gas chromatograph or other suitable analytical instrument. The plunger is then depressed again to expose the fiber and the organic analytes on the fiber are thermally desorbed and analyzed. The fiber remains in the analytical instrument during the analysis. When the analysis has been completed, the plunger is moved to the withdrawn position and the syringe is removed from the injection port. Various injection ports are suitable such as the "split-splitless" type or the "on-column" type.

While various types of syringes will be suitable, a HAMILTON 7000 (a trade mark) series syringe has been found to be suitable. The syringe facilitates convenient operation of the solid phase microextraction process and protects the fiber from damage during the introduction into a sample bottle or into an injector of an analytical instrument or even during storage. The length of the fiber depends on the injector of the analytical instrument with which the fiber will be used.

In addition to the improved convenience of the present device and method, the method differs significantly in the extraction part of the process compared to the prior art solid phase extraction process using cartridges. The extraction process embodying the present invention does not require prior sampling of aqueous material since in-vivo or in-vitro sampling can be conveniently performed. The microextractor can be directly inserted into the fluid stream. The simple geometry of the fiber eliminates clogging caused by particle matter present in the samples. Also, due to the small size of the fiber, not all of the organic compounds are extracted but rather the equilibrium described by the partition coefficient between the water and organic stationary phase for a given analyte is established. Therefore, the solid phase microextraction method embodying the present invention can be made selective by appropriate choice of a specifically designed organic phase. The partitioning between the aqueous phase and the organic coating can be described through the distribution constant, K:

$$K = \frac{C_s}{C_{aq}} \quad (1)$$

where $C_s$ is the concentration in the stationary phase and $C_{aq}$ is the concentration in the water. The partition ratio, k′, is therefore:

$$k' = \frac{C_s V_s}{C_{aq} V_{aq}} = \frac{n_s}{n_{aq}} = K \frac{V_s}{V_{aq}} \quad (2)$$

where $n_s$ and $n_{aq}$ are the number of moles in the stationary and aqueous phases, respectively, and $V_s$ and $V_{aq}$ are the volumes of the respective phases. Rearranging Eqn. 2 yields:

$$ns = K \frac{V_s n_{aq}}{V_{aq}} \quad (3)$$

substituting $C_{aq} V_{aq}$ for $n_{aq}$ results in:

$$n_s = K V_s C_{aq} = A C_{aq} \quad (4)$$

where $A = K V_s$.

A linear relationship between concentration of analytes in aqueous samples and detector response is expected based upon the relationship in equation (4). The slope of the linearity curve can be used to determine the partition coefficient for a given analyte if the volume of the stationary phase is known. Furthermore, the sensitivity of the fiber can be adjusted by changing the volume (thickness or area) of the stationary phase.

The linear dynamic range of the method typically extends several orders of magnitude for coatings similar to chromatographic stationary phase materials. The limit of quantization depends on the partition coefficient and the thickness of the coating and can be as low as a few ppT (parts per trillion), which was obtained for chlorinated solvents. In this case the amount of the solvents extracted by a thick polyimide coating from a water sample is about 30 pg per component at a 1μg/L concentration. This amount ensures not only ECD detection but will allow mass spectrometric identification and quantization.

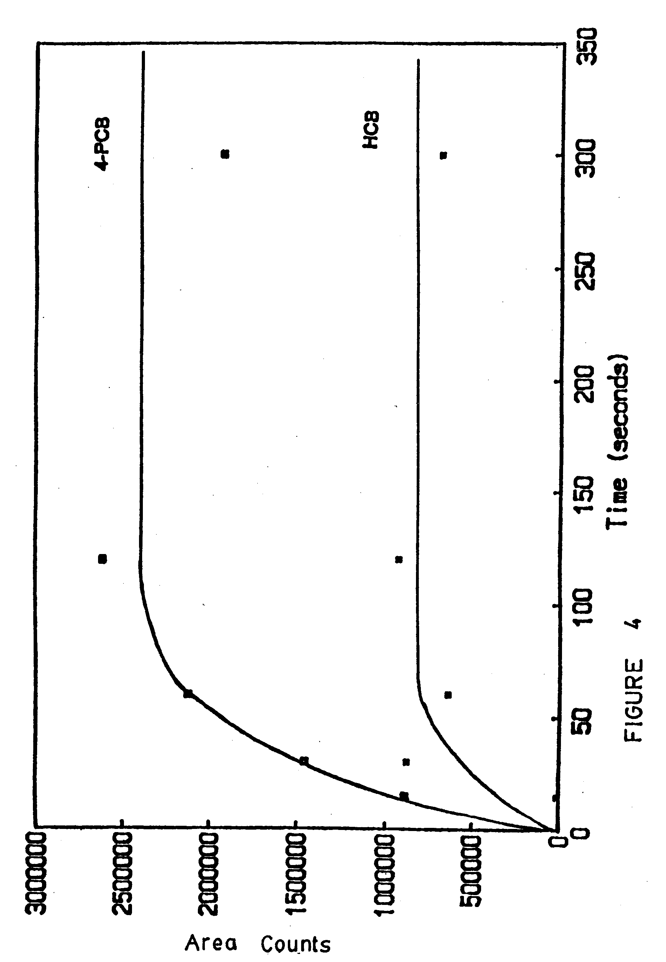

The dynamics of the extraction process is illustrated on Figure 4 which shows an example of a typical relationship between the amount of analyte adsorbed onto the microextractor (peak area) versus the extraction time, which corresponds to the exposure time of the fiber to the water matrix sample. Initially, the amount of analyte adsorbed by the stationary phase increases with the increase in extraction time. This trend is continued until the point of steady state is achieved which causes the relationship to level off. This situation indicates the state of equilibrium between the concentration of the analyte in the stationary phase and in the water matrix sample and defines optimum extraction time. According to Figure 3, optimum extraction time for uncoated fiber (about .1 μm film of silica gel) and PCBs as analytes is about one minute.

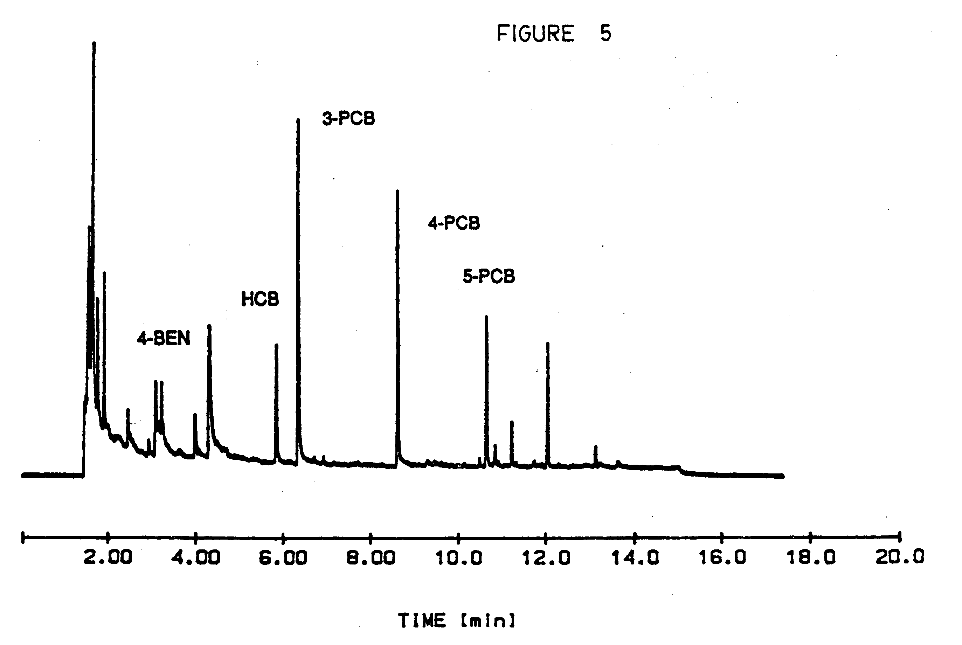

Figure 5 illustrates the chromatogram corresponding to a PCB mixture in water extracted and analyzed by the solid phase microextraction method. Peak tailing is larger for the more volatile compounds than the heavier, later eluting components. This is an artifact of thermal focussing that occurs when the analytes are volatilized at 300° C and transferred to a 150° C oven. The heavier compounds benefit from thermal focussing, but the oven is at too high a temperature to allow focussing of the more volatile compounds. The tailing can be alleviated by using a cryogenically cooled oven to improve focussing.

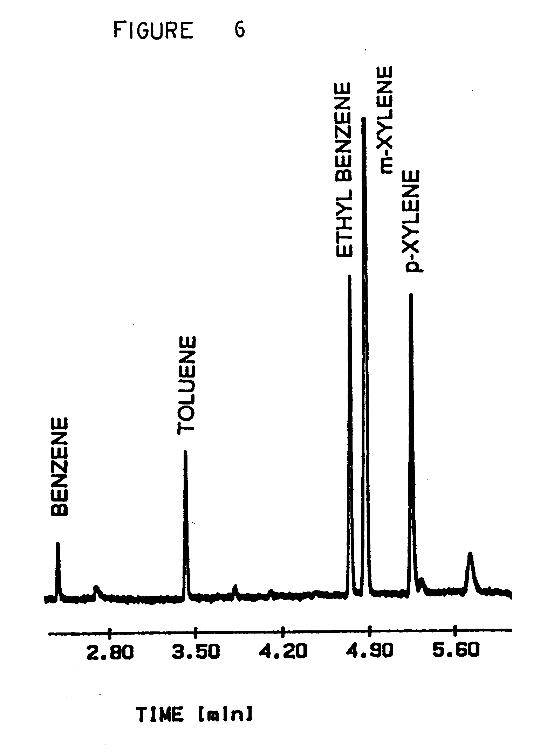

An uncoated fiber can also be used to adsorb benzene, toluene, ethyl benzene and xylenes (BTEX) from aqueous solutions. For this separation (Figure 6), a flame ionization detector (FID) was used, illustrating that a sufficient quantity was adsorbed for FID detection. This expands the general applicability of the fiber as FID detectors are somewhat easier to operate and maintain than ECD detectors.- The extraction efficiency in this case is sufficiently high to deplete significantly the analyte after 2 to 3 injections if a small volume of aqueous material (1 to 2 mL) is sampled. A larger sample volume (100 mL) is thus recommended if multiple injections are necessary.

Moderate levels of organic interferences and variation in ionic strength of aqueous solution do not significantly change the extraction equilibria. However, large amounts of organic solvent could be added intentionally to introduce partitioning selectivity, as is commonly done in liquid chromatography.

The fiber method has great potential for the analysis of highly sorptive compounds that can be difficult to sample without loss of analyte. Losses to storage bottles and transfer lines could potentially be eliminated by sampling in situ and analyzing the fiber in the field using portable gas chromatograph instrumentation. The device and method of the present invention can utilize a mechanical device such as an autosampler. The autosampler can be programmed to operate the plunger at the appropriate time to contact the carrier and to insert the syringe and the fiber into the injection port of the analytical instrument. The autosampler has an advantage over manual extraction and analysis in that the contact time and the length of the fiber in the carrier as well as in the instrument can be maintained constant. A VARIAN 3500 gas chromatograph and a VARIAN 8100 autosampler has been found to be suitable.

Possible applications of this technique include sampling of both surface and groundwater samples, either in situ or in the laboratory. It could potentially be used in on-line process applications or clinical analysis. Both of these applications benefit from the simplified sample preparation. The coating can be designed for either a broad scan of the organic contaminants (non-selective fiber coating) or selective sampling. This method, when combined with laser desorption, could reduce the sample extraction and analysis to a fraction of a minute. In this technique the optical fiber is used as a light guide. In a variation embodying the invention, the syringe could have a laser source affixed thereto with activation means and coupling optics to focus light onto the fiber which will transmit the light to a free end thereof to desorb the components thereon. Curie point heating and microwave desorption are alternative desorption methods. The fiber also shows promise as a method of studying the adsorption properties of polymers and for obtaining information about partitioning in liquid chromatographic systems.

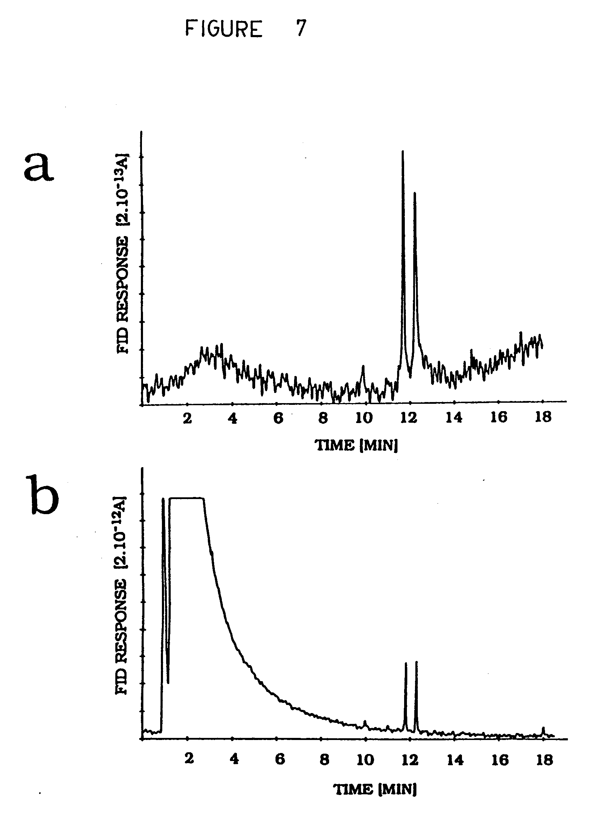

Figure 7 illustrates the advantages of the method embodying the present invention compared to the prior art solvent procedure. The chromatogram from Figure 7a corresponds to silica fiber techniques using C-18 coating and Figure 7b to liquid-liquid extraction with chloroform. In both cases the same effluent from a sewage treatment plant was analyzed under the same chromatographic conditions. Results are similar, however the total extraction time was about an hour for the solvent method and two minutes for the fused silica fiber technique. The chromatogram for Figure 7b shows the presence of the solvents used in the liquid-liquid extraction. The solid phase microextraction device facilitates easy sampling in the field. In addition, when organic solvents are used in the preparation step, the corresponding large peak together with possible impurities can mask volatile analytes (Figure 7b).

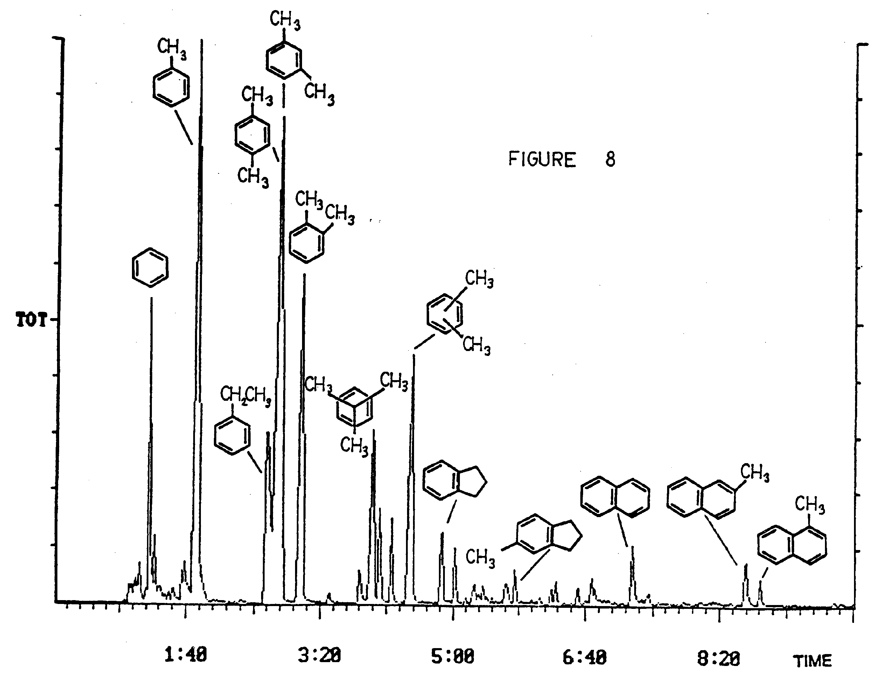

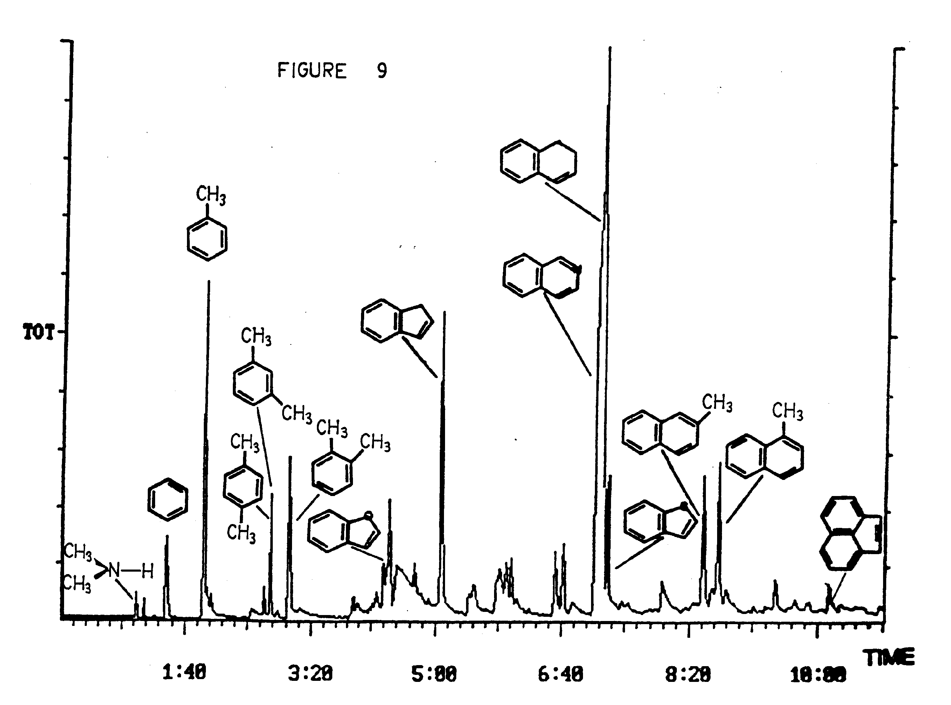

In Figure 8, there is shown a chromatograph for the extraction of gasoline components from water using a silicone coated fiber. In Figure 9, there is shown a chromatograph for the extraction of organics from coal gasification waste water using a silicone coated fiber. Both analyses and identifications for Figures 8 and 9 have been done using a mass spectrometry detector.

The device and method embodying the present invention can also be used for extraction and analysis of gases and for supercritical fluids as well. The method is not limited to analysis of organic analytes but also for inorganic ions by using ion-exchange materials located on the fiber surface. In addition to thermal desorption by direct heating, laser desorption or conductive heating, for example, microwave desorption or Curie point magnetic hysteresis method could be used. Various fibers will be suitable depending on the use that is being made of the present invention. For example, fused silica, graphite fibers, fibers constructed with solid polymeric materials and even metal wires can be used as fibers and the fibers can be coated with various materials or uncoated. Some suggested coatings are CARBOWAX (a trade mark), octadecyltrichlorosilane, polymethylvinylchlorosilane, liquid crystalline polyacrylates, silicone, polyimide and grafted self-assembled monolayers. Fibers coated with these coatings are stored under nitrogen or helium to prevent absorption of the volatile organics present in air. The coatings can be organic or inorganic, for example, fused silica surface.

In addition to having coating located on an outer surface of a solid fiber, coating could be located on an inner surface of a hollow fiber. Coating could also be located on the packing material used with the fiber. In addition to direct extraction, the method of the present application could be performed with prior activation using organic solvents by using the optional inlet 26 on the syringe. The analytical instrument used with the method embodying the present invention can also be varied. For example, a gas chromatograph, a liquid chromatograph or a supercritical fluid chromatograph could be used. Other analytical methods such as flow injection analysis, mass spectrometry, atomic absorption or emission including inductively coupled plasma technique could be used.

In addition to analyzing for environmental contaminants, the method and device of the present invention can be used to monitor or measure the components in industrial process streams. The present invention can also be used to study properties of coatings, for example, absorption, deterioration rates and diffusion coefficients.

Numerous other variations, within the scope of the attached claims, will be readily apparent to those skilled in the art.

## Claims

1. A device for carrying out solid phase microextraction with components contained in a fluid carrier, said device being characterized by, in combination, a fiber (6) and a housing surrounding said fiber, said housing containing access means (26, 28) so that said carrier and components can be brought into contact with said fiber.

2. A device as claimed in Claim 1 wherein the housing is a syringe (4) having a barrel (8) and plunger (10) slidable within said barrel, said plunger having a handle (12) extending from one end (14) of said barrel, said fiber being mounted within said syringe so that said fiber will move longitudinally within said syringe as said plunger is moved within said barrel.

3. A device as claimed in Claim 2 wherein a hollow needle (18) is connected to said barrel at an end (16) opposite to said plunger, said needle containing said fiber, said fiber at least partially extending beyond a free end (28) of said needle when said plunger is depressed within said barrel and said fiber being located within said needle when said plunger is withdrawn relative to said barrel.

4. A device as claimed in Claim 3 wherein the fiber is partially contained in a metal sleeve (24) within said syringe, said sleeve protecting the fiber from damage, said fiber extending from a plunger end of said barrel through said barrel and into said needle, said fiber having retention means (22) thereon, said retention means being located at said plunger end of said barrel.

5. A device as claimed in any one of Claims 1, 2 or 3 wherein the fiber is hollow and the carrier and components contact an interior surface of said fiber.

6. A device as claimed in Claim 3 wherein the fiber is hollow and the needle contains suitable openings so that said carrier and components can contact an interior of said fiber.

7. A device as claimed in any one of Claims 1, 2 or 6 wherein the fiber has a coating thereon on a surface of the fiber that is contacted by said carrier and components.

8. A device as claimed in Claim 6 wherein the interior of the fiber is packed with a sorbent material.

9. A device as claimed in any one of Claims 1, 2 or 3 wherein an outer surface of the fiber is packed with sorbent material.

10. A device as claimed in any one of Claims 1, 2 or 3 wherein said fiber is solid.

11. A device as claimed in any one of Claims 1, 2 or 3 wherein said fiber is solid and the fiber has a coating thereon on a surface of the fiber that is contacted by said carrier and components.

12. A device as claimed in any one of Claims 2, 3 or 4 wherein the syringe contains an inlet to receive one of activation solvent and the carrier.

13. A device as claimed in any one of Claims 1, 3 or 6 wherein the fiber has a coating thereon selected from the group of carbowax, silicone, polyimide, octadecyltrichlorosilane, polymethylvinylchlorosilane, liquid crystalline polyacrylates, grafted self-assembled monolayers and inorganic coatings.

**14.** A device as claimed in any one of Claims 1, 3 or 6 wherein the fiber is selected from the group of fused silica fibers, graphite fibers, fibers made from solid polymeric materials and fibers made from wires of various metals.

**15.** A device as claimed in any one of Claims 2, 3 or 4 wherein the syringe is connected to an autosampler, said autosampler being programmable to manipulate the plunger and to carry out extraction and analysis.

**16.** A device as claimed in any one of Claims 2, 3 or 4 wherein the syringe has a laser source affixed to it, with activation means for said laser, light from said laser desorbing the fiber into an analytical instrument.

**17.** A method of carrying out solid phase microextraction and analysis with components contained in a carrier using a fiber (6) contained in a housing, said housing having access means so that said carrier can be brought into contact with said fiber, said method being characterized by contacting said fiber with said carrier for a sufficient time to allow extraction to occur, ending said contact and placing the fiber in a suitable analytical instrument in such a manner that desorption occurs with respect to at least one component on said fiber.

**18.** A method of carrying out solid phase microextraction and analysis as claimed in Claim 17 wherein the housing is a syringe (4), said syringe having a barrel (8) and a plunger (10) slidable within said barrel, said plunger having a handle (12) extending from one end (14) of said barrel, with a hollow needle (18) extending from the other end (28) of said barrel, said fiber being mounted within said syringe so that the fiber is located within the needle when the plunger is in a withdrawn position, said fiber extending beyond a free end of said needle when the plunger is in a depressed position, said method being characterized by depressing said plunger to expose said fiber, placing said fiber in contact with said carrier and said components of said carrier for a sufficient time to allow extraction to occur between said fiber and said components, moving said plunger to a withdrawn position to cause the fiber to return to an interior of said needle, subsequently placing the needle into an injection port of an analytical instrument, depressing said plunger to expose said fiber so that desorption will occur, subsequently moving said plunger to the withdrawn position and removing said needle from said injection port.

**19.** A method of carrying out solid phase microextraction and analysis as claimed in Claim 18 wherein samples are analyzed and the samples are contained in containers having a septum for receiving the needle of the syringe, said method utilizing an analytical instrument having an injection port containing a septum for receiving said needle, said method being characterized by the steps of commencing with the plunger in a withdrawn position and the fiber located within said needle, placing said needle through the septum of a container containing a sample to be analyzed, depressing said plunger to cause said fiber to extend beyond said needle into contact with said sample, continuing said contact for a sufficient time to allow microextraction to occur, moving the plunger to the withdrawn position, removing the syringe from said container, inserting the needle of said syringe through the septum of said injection port of said analytical instrument, depressing the plunger to expose said fiber to said instrument in such a manner that desorption will occur between at least one component on said fiber and said instrument, subsequently moving the plunger to the withdrawn position and withdrawing said needle from said instrument.

**20.** A method as claimed in any one of Claims 17, 18 or 19 wherein the carrier being analyzed is selected from the group of water, liquid, gas and supercritical fluids.

**21.** A method as claimed in any one of Claims 17, 18 or 19 wherein the extraction and analysis are carried out automatically through the use of an autosampler.

**22.** A method as claimed in any one of Claims 18 or 19 wherein the device has a laser source on the syringe, with activating means and coupling optics to focus laser light onto the fiber, said fiber transmitting the light to a free end of the fiber where the components are located thereby desorbing the components.

**23.** A method of carrying out solid phase microextraction and analysis, with components contained in a carrier, using a fiber (6), said method being characterized by placing said fiber in contact with said carrier containing said components for a sufficient period of time for extraction to occur, subsequently removing said fiber from said carrier and placing the fiber into a suitable analytical instrument and carrying out thermal desorption with respect to at least one component on said fiber.

**24.** A method as claimed in Claim 23 wherein the carrier is stirred while the fiber is placed into it.

25. A method as claimed in Claim 23 wherein the fiber is placed in contact with said carrier for approximately two minutes.

**Patentansprüche**

1. Vorrichtung zum Ausführen Festphasenmikroextraktion der mit in einem Trägerfluid enthaltenen Bestandteilen, gekennzeichnet durch eine Kombination aus einer Faser (6) und einem die Faser umgebenden Gehäuse, wobei das Gehäuse mit Zugängen (26, 28) versehen ist, durch die das genannte Trägerfluid und die Bestandteile mit der Faser in Kontakt gebracht werden können.

2. Vorrichtung nach Anspruch 1, bei der das Gehäuse aus einer Spritze (4) mit einem Zylinder (8) und einem in dem Zylinder verschiebbaren Kolben (10) besteht, wobei der Kolben mit einem sich von einem Ende (14) des Zylinders erstreckenden Griff (12) versehen ist und die Faser so in der Spritze angeordnet ist, daß sie sich in Längsrichtung in der Spritze bewegt, wenn der Kolben in dem Zylinder bewegt wird.

3. Vorrichtung nach Anspruch 2, bei der eine Hohlnadel (18) an einem dem genannten Kolben gegenüberliegenden Ende (16) mit dem genannten Zylinder verbunden ist, wobei die Nadel die genannte Faser enthält, wobei sich die Faser mindestens teilweise über ein freies Ende (28) der Nadel hinauserstreckt, wenn der Kolben in den Zylinder gedrückt wird, und die Faser in der Nadel liegt, wenn der Kolben gegenüber dem Zylinder zurückgezogen wird.

4. Vorrichtung nach Anspruch 3, bei der die Faser teilweise in einer Metallhülse (24) in der genannten Spritze liegt, wobei die Hülse die Faser vor Beschädigung schützt, wobei sich die Faser von einem Kolbenende des Zylinders durch den Zylinder und in die Nadel hineinerstreckt, und wobei die genannte Faser mit Haltemitteln (22) versehen ist, die sich an dem Kolbenende des Zylinders befinden.

5. Vorrichtung nach einem der Ansprüche 1, 2 oder 3, bei der die Faser hohl ausgebildet ist und das Trägerfluid und die Bestandteile mit einer Innenfläche der Faser in Kontakt stehen.

6. Vorrichtung nach Anspruch 3, bei der die Faser hohl ausgebildet ist und die Nadel mit einer ausreichenden Anzahl von Öffnungen versehen ist, so daß das genannte Trägerfluid und die Bestandteile in das Innere der Faser gelangen können.

7. Vorrichtung nach einem der Ansprüche 1, 2 oder 6, bei der die Faser auf ihrer Oberfläche mit einer Beschichtung versehen ist, mit der das Trägerfluid und die Bestandteile in Kontakt treten.

8. Vorrichtung nach Anspruch 6, bei der das Faserinnere mit einem Sorptionsmittel versehen ist.

9. Vorrichtung nach einem der Ansprüche 1, 2 oder 3, bei der eine Außenfläche der Faser mit Sorptionsmittel versehen ist.

10. Vorrichtung nach einem der Ansprüche 1, 2 oder 3, bei der die genannte Faser fest ist.

11. Vorrichtung nach einem der Ansprüche 1, 2 oder 3, bei der die genannte Faser fest ist und die Faser auf ihrer Oberfläche mit einer Beschichtung versehen ist, mit der das Trägerfluid und die Bestandteile in Kontakt treten.

12. Vorrichtung nach einem der Ansprüche 2, 3 oder 4, bei der die Spritze einen Eingang zur Aufnahme eines Aktivierungslösungsmittels und des Trägerfluids aufweist.

13. Vorrichtung nach einem der Ansprüche 1, 3 oder 6, bei der die Faser mit einer Beschichtung versehen ist, die aus der Gruppe Carbowax, Silikon, Polyimid, Octadecyltrichlorosilan, Polymethylvinylchlorsilan, flüssigkristalline Polyacrylate, gepfropften selbstorganisiserten monomolekularen Schichten und anorganischen Beschichtungsmaterialen ausgewählt ist.

14. Vorrichtung nach einem der Ansprüche 1, 3 oder 6, bei der die Faser aus der Gruppe Schmelzquarzfasern, Graphitfasern aus festem Polymermaterial bestehende Fasern und aus Fasern aus Drähten verschiedener Materialien ausgewählt ist.

15. Vorrichtung nach einem der Ansprüche 2, 3 oder 4, bei der die Spritze an einen automatischen Probennehmer angeschlossen ist, der zur Manipulation des Kolbens und zur Ausführung der Extraktion und Analyse programmierbar ist.

16. Vorrichtung nach einem der Ansprüche 2, 3 oder 4, bei der die Spritze mit einer an ihr befestigten Laserquelle versehen ist, mit Aktivierungsmitteln für den Laser, wobei das von dem Laser ausgehende Licht die Faser in ein Analyseinstrument desorbiert.

**17.** Verfahren zum Ausführen der Festphasenmikroextraktion und Analyse mit in einem Trägerfluid befindlichen Bestandteilen unter Verwendung einer in einem Gehäuse befindlichen Faser (6), wobei das Gehäuse mit Zugangsmitteln versehen ist, so daß das Trägerfluid mit der Faser in Kontakt gebracht werden kann, gekennzeichnet durch Inkontaktbringen der Faser mit dem Trägerfluid während einer Dauer, die ausreichend lang ist, daß die Extraktion erfolgen kann, Beenden des Kontaktes und Plazieren der Faser in ein geeignetes Analyseinstrument in der Weise, so daß es zur Desorption mindestens eines Bestandteiles auf der genannten Faser kommt.

**18.** Verfahren zum Ausführen der Festphasenmikroextraktion und Analyse nach Anspruch 17, nach dem das Gehäuse eine Spritze (4) ist, wobei die Spritze einen Zylinder (8) und einen in dem Zylinder beweglichen Kolben (10), wobei der Kolben mit einem sich von einem Ende (14) des Zylinders erstreckenden Griff (12) versehen ist, und eine sich vom anderen Ende (28) des Zylinders erstreckenden Hohlnadel (18) aufweist, wobei die genannte Faser so in der Spritze angeordnet ist, daß sich die Faser in der Nadel befindet, wenn sich der Kolben in einer zurückgezogenen Stellung befindet, und sich die Faser über ein freies Ende der Nadel hinaus erstreckt, wenn sich der Kolben in einer gedrückten Stellung befindet, gekennzeichnet durch Drücken des Kolbens, um die Faser herauszuschieben, Plazieren der Faser in Kontakt mit dem Trägerfluid und den Bestandteilen des Trägerfluides während einer Dauer, die zur Extraktion zwischen der Faser und den Bestandteilen ausreicht, Bewegen des Kolbens in eine zurückgezogene Stellung, um die Faser zur Rückkehr in das Innere der Nadel zu veranlassen, und nachfolgendes Einbringen der Nadel in eine Einspritzöffnung eines Analyseinstrumentes, Drücken des Kolbens, um die Faser so herauszuschieben, daß die Desorption erfolgt, nachfolgendes Bewegen des Kolbens in die zurückgezogene Stellung und Entfernen der Nadel aus der genannten Einspritzöffnung.

**19.** Verfahren zum Ausführen der Festphasenmikroextraktion und Analyse nach Anspruch 18, nach dem Proben analysiert werden und sich die Proben in Behältern mit einem Septum zur Aufnahme der Nadel der Spritze befinden, wobei nach dem Verfahren ein Analyseinstrument mit einer Einspritzöffnung mit einem Septum zur Aufnahme der genannten Nadel verwendet wird, gekennzeichnet durch die Schritte des Beginnens mit dem Kolben in einer zurückgezogenen Stellung und der sich in der Nadel befindenden Faser, des Verbringens der Nadel durch das Septum eines Behälters, der die zu analysierende Probe enthält, des Drückens des Kolbens, um die Faser aus der Nadel heraus und in Kontakt mit der Probe zu bringen, des Fortsetzens des Kontaktes während einer Dauer, die ausreicht, das Stattfinden der Mikroextraktion zu erlauben, des Bewegens des Kolbens in die zurückgezogene Stellung, des Entfernens der Spritze aus dem Behälter, des Einsetzens der Nadel der Spritze durch das Septum der Einspritzöffnung des Analyseinstrumentes, des Drückens des Kolbens, um die Faser in der Weise zu dem genannten Instrument freizulegen, daß es zwischen mindestens einem Bestandteil auf der genannten Faser und dem Instrument zur Desorption kommt, des anschließenden Bewegens des Kolbens in die zurückgezogene Stellung und des Zurückziehens der Nadel aus dem Instrument.

**20.** Verfahren nach einem der Ansprüche 17, 18 oder 19, nach dem das analysierte Trägerfluid aus der Gruppe Wasser, Flüssigkeit, Gas und aus überkritischen Fluiden ausgewählt wird.

**21.** Verfahren nach einem der Ansprüche 17, 18 oder 19, nach dem die Extraktion und Analyse mit einem automatischen Probennehmer automatisch durchgeführt werden.

**22.** Verfahren nach einem der Ansprüche 18 oder 19, nach dem die Vorrichtung mit einer Laserquelle an der Spritze mit Aktivierungsmitteln und mit einer optischen Kupplung versehen ist, um das Laserlicht auf die Faser zu fokussieren, wobei die Faser das Licht auf ein freies Ende der Faser überträgt, an dem sich die Bestandteile befinden, wodurch die Bestandteile desorbiert werden.

**23.** Verfahren zum Ausführen der Festphasenmikroextraktion und Analyse mit in einem Trägerfluid enthaltenen Bestandteilen, bei dem eine Faser (6) verwendet wird, gekennzeichnet durch Inkontaktbringen der Faser mit dem die Bestandteile enthaltenden Trägerfluid während einer Dauer, die ausreicht, eine Extraktion stattfinden zu lassen, und nachfolgendes Entfernen der Faser aus dem Trägerfluid und Einbringen der Faser in ein geeignetes Analyseinstrument und Durchführen der thermischen Desorption in bezug auf mindestens einen Bestandteil auf der Faser.

**24.** Verfahren nach Anspruch 23, nach dem das Trägerfluid umgerührt wird, während die Faser darin eingebracht wird.

**25.** Verfahren nach Anspruch 23, nach dem die Faser für eine Dauer von etwa 2 Minuten mit dem genannten Trägerfluid in Kontakt gebracht wird.

## Revendications

1. Dispositif pour réaliser une micro-extraction en phase solide avec des composants contenus dans un porteur fluide, ledit dispositif étant caractérisé par, en combinaison , une fibre (6) et un logement entourant ladite fibre, ledit logement contenant des moyens d'accès (26, 28) de sorte que ledit porteur et lesdits composants peuvent être amenés en contact avec ladite fibre.

2. Dispositif selon la revendication 1, dans lequel le logement est une seringue (4) ayant un barillet et un piston (10) coulissant dans ledit barillet, ledit piston ayant un manche (12) s'étendent à partir d'une extrémité (14) dudit barillet, ladite fibre étant montée dans ladite seringue de sorte que ladite fibre se déplace longitudinalement dans ladite seringue à mesure que le piston est déplacé à l'intérieur dudit barillet.

3. Dispositif selon la revendication 2, dans lequel une aiguille creuse (18) est fixée audit barillet à une extrémité (16) opposée audit piston, ladite aiguille contenant ladite fibre, ladite fibre s'étendant au moins partiellement au-delà d'une extrémité libre (28) de ladite aiguille lorsque ledit piston est enfoncé dans ledit barillet et ladite fibre étant située dans ladite aiguille lorsque ledit piston est retiré par rapport audit barillet.

4. Dispositif selon la revendication 3, dans lequel la fibre est contenue partiellement dans un manchon métallique (24) sur ladite seringue, ledit manchon protégeant la fibre des dommages, ladite fibre s'étendant à partir d'une extrémité du piston dudit barillet au travers dudit barillet et dans ladite aiguille, ladite fibre ayant des moyens de maintien (22), lesdits moyens de maintien étant situés à ladite extrémité du piston dudit barillet.

5. Dispositif selon l'une quelconque des revendications 1 à 3, dans lequel la fibre est creuse et le porteur et les composants sont en contact avec une surface intérieure de ladite fibre.

6. Dispositif selon la revendication 3, dans lequel la fibre est creuse et l'aiguille contient des ouvertures appropriées de sorte que ledit porteur et les composants peuvent établir un contact avec un intérieur de ladite fibre.

7. Dispositif selon l'une quelconque des revendications 1, 2 ou 6, dans lequel la fibre a un revêtement sur une surface de la fibre qui est en contact avec ledit porteur et lesdits composants.

8. Dispositif selon la revendication 6, dans lequel l'intérieur de la fibre est garni avec un agent de sorption.

9. Dispositif selon l'une quelconque des revendications 1 à 3, dans lequel une surface externe de la fibre est garnie avec un agent de sorption.

10. Dispositif selon l'une quelconque des revendications 1 à 3, dans lequel ladite fibre est solide.

11. Dispositif selon l'une quelconque des revendications 1 à 3, dans lequel ladite fibre est solide et la fibre a un revêtement sur une surface de la fibre qui est en contact avec ledit porteur et lesdits composants.

12. Dispositif selon l'une quelconque des revendications 2, 3 ou 4, dans lequel la seringue contient une entrée pour recevoir un des deux d'un solvant d'activation et du porteur.

13. Dispositif selon l'une quelconque des revendications 1, 3 ou 6, dans lequel la fibre a un revêtement choisi dans le groupe constitué de cire de carbone, silicone, polyimide, octadecyltrichlorosilane, polyméthylvinylchlorosilane, de polyacrylates cristallins liquides, de monocouches auto-assemblées greffées et de revêtements inorganiques.

14. Dispositif selon l'une quelconque des revendications 1, 3 ou 6, dans lequel la fibre est choisie dans le groupe constitué de fibres de verre de silice, fibres de graphites, fibres fabriquées à partir de matériaux polymères solides et des fibres fabriquées à partir de fils de divers métaux.

15. Dispositif selon l'une quelconque des revendications 2, 3 ou 4, dans lequel la seringue est connectée à un échantillonneur automatique, ledit échantillonneur automatique étant programmable pour manipuler le piston et effectuer l'extraction et les analyses.

16. Dispositif selon l'une quelconque des revendications 2, 3 ou 4, dans lequel la seringue a une source laser rattachée à elle, avec des moyens d'activation pour ledit laser, la lumière dudit laser désorbant la fibre dans un instrument d'analyse.

17. Procédé pour réaliser une micro-extraction en phase solide et une analyse avec des composants contenus dans un porteur en utilisant une fibre (6) contenue dans un logement, ledit logement ayant des moyens d'accès de sorte que ledit porteur peut être amené en contact de ladite fibre, ledit procédé étant caractérisé par : mettre en contact ladite fibre et ledit porteur pendant un temps suffisant pour permettre à l'extraction de se produire, rompre ledit contact et placer la fibre dans un instrument d'analyse approprié de telle manière qu'une désorption se produit par rapport à au moins un composant sur ladite fibre.

18. Procédé pour réaliser une micro-extraction en phase solide et une analyse selon la revendication 17, dans lequel le logement est une seringue (4), ladite seringue ayant un barillet (8) et un piston (10) coulissant dans ledit barillet, ledit piston ayant un manche (12) s'étendant à partir d'une extrémité (14) dudit barillet, avec une aiguille creuse (18) s'étendant à partir de l'autre extrémité (28) dudit barillet, ladite fibre étant montée dans ladite seringue de sorte que la fibre est placée dans l'aiguille lorsque le piston est dans une position retirée, ladite fibre s'étendant au-dlà d'une extrémité libre de ladite aiguille lorsque le piston est dans une position enfoncée, ledit procédé étant caractérisé par : enfoncer ledit piston pour exposer ladite fibre, placer ladite fibre en contact dudit porteur et desdits composants dudit porteur pendant un temps suffisant pour permettre à l'extraction de se produire entre ladite fibre et lesdits composants, déplacer ledit piston vers une position retirée pour faire retourner la fibre vers un intérieur de ladite aiguille, puis placer l'aiguille dans un orifice d'injection d'un instrument d'analyse, enfoncer ledit piston pour exposer ladite fibre de sorte qu'une désorption se produise, puis déplacer ledit piston vers la position retirée et retirer ladite aiguille dudit orifice d'injection.

19. Procédé pour réaliser une micro-extraction en phase solide et une analyse selon la revendication 18, dans lequel des échantillons sont analysés et les échantillons sont contenus dans des récipients ayant une cloison pour recevoir l'aiguille de la seringue, ledit procédé utilisant un instrument d'analyse ayant un orifice d'injection contenant une cloison pour recevoir ladite aiguille, ledit procédé étant caractérisé par les étapes consistant à commencer avec le piston dans une position retirée et la fibre située dans ladite aiguille, placer ladite aiguille à travers la cloison d'un récipient contenant un échantillon à analyser, enfoncer ledit piston pour étendre ladite fibre au-delà de ladite aiguille en contact avec ledit échantillon, continuer ledit contact pendant un temps suffisant pour permettre à la micro-extraction de se produire, déplacer le piston vers la position retirée, retirer la seringue dudit récipient, insérer l'aiguille de ladite seringue à travers la cloison dudit orifice d'injection dudit instrument d'analyse, enfoncer le piston pour exposer ladite fibre audit instrument de telle manière qu'une désorption se produise entre au moins un composant sur ladite fibre et ledit instrument, puis déplacer ledit piston vers la position retirée et retirer l'aiguille dudit instrument.

20. Procédé selon l'une quelconque des revendications 17 à 19, dans lequel le porteur qui est analysé est choisi dans le groupe constitué d'eau, de liquide, de gaz et de fluides supercritiques.

21. Procédé selon l'une quelconque des revendications 17 à 19, dans lequel l'extraction et l'analyse sont effectuées automatiquement en utilisant un échantillonneur automatique.

22. Procédé selon l'une quelconque des revendications 18 ou 19, dans lequel le dispositif a une source laser sur la seringue, avec des moyens d'activation et des optiques de couplage pour focaliser la lumière laser sur la fibre, ladite fibre transmettant la lumière vers une extrémité libre de la fibre où les composants sont situés, désorbant ainsi les composants.

23. Procédé pour réaliser une micro-extraction en phase solide et une analyse, avec des composants contenus dans un porteur, en utilisant une fibre (6), ledit procédé étant caractérisé par : placer ladite fibre en contact dudit porteur contenant lesdits composants pendant une période de temps suffisante pour que l'extraction se produise, puis retirer ladite fibre dudit porteur et placer la fibre dans un instrument d'analyse approprié et effectuer une désorption thermique par rapport à au moins un composant sur ladite fibre.

24. Procédé selon la revendication 23, dans lequel on remue le porteur alors que la fibre est placée dedans.

25. Procédé selon la revendication 23, dans lequel la fibre est placée en contact dudit porteur pendant deux minutes approximativement.

FIGURE 1

FIGURE 2

FIGURE 3

FIGURE 4

EP 0 523 092 B1

FIGURE 5

FIGURE 6

FIGURE 7

FIGURE 8

FIGURE 9

EP 0 523 092 B1